# EUROPEAN PATENT APPLICATION

(11) **EP 4 299 715 A1**
(43) Date of publication of application: **03.01.2024**
(21) Application number: 21934457.9
(22) Date of filing: 28.09.2021
(51) Int. Cl.: C12M 3/00, C12M 1/04, C12M 1/00

(54) **CULTURE DEVICE AND CULTURE METHOD**

(30) Priority: 30.03.2021 CN 202110341856; 30.03.2021 CN 202110341842; 21.04.2021 CN 202110430174
(71) Applicant: Shanghai Ruiyu Biotech Co. Ltd., Shanghai 201615 (CN)
(72) Inventor: WANG, Xuan, Shanghai 201615 (CN)
(74) Representative: Wang, Bo
(86) International application number: PCT/CN2021/121430
(87) International publication number: WO 2022/205820

(57) **Abstract**

The present disclosure discloses a culture device and method. The culture device includes a substrate, wherein a surface of the substrate includes a plurality of culture regions. Each of the plurality of culture regions are configured to adhere a culture. An adhesion affinity of an interior of each of the plurality of culture regions is greater than an adhesion affinity of an exterior of each of the plurality of culture regions. The present disclosure further discloses a culture method for biological culture using the culture device.

## Description

### CROSS-REFERENCE TO RELATED APPLICATIONS

This application claims priority of Chinese Patent Application No. 202110341856.5, filed on March 30, 2021, Chinese Patent Application No.202110341842.3, filed on March 30, 2021, and Chinese Patent Application No. 202110430174.1, filed on April 21, 2021, the entire contents of each of which are incorporated herein by reference.

### TECHNICAL FIELD

The present disclosure relates to the field of biological culture technology, and in particular, to culture devices and methods.

### BACKGROUND

With the continuous development of modern society, in vitro culture techniques for biological entities (e.g., cells, bacteria, viruses, etc.) have been paid more and more attention. Taking cells as an example, cell culture is a crucial and commonly used technique in cell biology research. Through the cell culture, a large quantity of cells may be obtained, enabling the study of signal transduction of the cells, synthesis and metabolism of the cells, cell growth and reproduction, etc.

### SUMMARY

Some embodiments of the present disclosure provide a culture device. The culture device includes a substrate, wherein a surface of the substrate includes a plurality of culture regions. Each of the plurality of culture regions are configured to adhere a culture. An adhesion affinity of an interior of each of the plurality of culture regions is greater than an adhesion affinity of an exterior of each of the plurality of culture regions.

In some embodiments, a roughness of the interior of each of the plurality of culture regions is different from a roughness of the exterior of each of the plurality of culture regions, such that the adhesion affinity of the interior of each of the plurality of culture regions to the culture is greater than the adhesion affinity of the exterior of each of the plurality of culture regions to the culture.

In some embodiments, the surface of the substrate is hydrophilic, and the roughness of the interior of each of the plurality of culture regions is greater than the roughness of the exterior of each of the plurality of culture regions.

In some embodiments, a polishing or etching operation is performed on the interior of each of the plurality of culture regions, such that the roughness of the interior of each of the plurality of culture regions is greater than the roughness of the exterior of each of the plurality of culture regions.

In some embodiments, the etching operation includes at least one of a soft etching operation, a laser etching operation, a plasma etching operation, an electron beam etching operation, or a chemical etching operation.

In some embodiments, a surface of the interior of each of the plurality of culture regions has a regular structure at submicron to nanometer level.

In some embodiments, the surface of the substrate is hydrophobic, the roughness of the interior of each of the plurality of culture regions is greater than the roughness of the exterior of each of the plurality of culture regions, and a surface of the interior of each of the plurality of culture regions has a regular structure at submicron to nanometer level.

In some embodiments, the surface of the substrate is hydrophobic, the roughness of the interior of each of the plurality of culture regions is less than the roughness of the exterior of each of the plurality of culture regions, and a surface of the exterior of each of the plurality of culture regions has an irregular structure.

In some embodiments, a polishing operation is performed on the exterior of each of the plurality of culture regions, such that the roughness of the interior of each of the plurality of culture regions is less than the roughness of the exterior of each of the plurality of culture regions.

In some embodiments, a surface of the interior of each of the plurality of culture regions has a regular structure at submicron to nanometer level, such that the adhesion affinity of the interior of each of the plurality of culture regions towards the culture is greater than the adhesion affinity of the exterior of each of the plurality of culture regions towards the culture.

In some embodiments, the regular structure at submicron to nanometer level include a plurality of micro-nano monomers arranged in an array, the plurality of micro-nano monomers including at least one of a micro-nano pillar, a micro-nano tube, a micro-nano cone, or a micro-nano wall.

In some embodiments, a diameter or a maximum width of each of the plurality of the micro-nano monomers is less than 1 micron.

In some embodiments, a head of each of the plurality of the micro-nano monomers has a mushroom-like shape.

In some embodiments, an etching operation is performed on the interior of each of the plurality of culture regions, such that the surface of the interior of each of the plurality of culture regions has the regular structure at submicron to nanometer level.

In some embodiments, the etching operation includes at least one of a soft etching operation, a laser etching operation, a plasma etching operation, an electron beam etching operation, or a chemical etching operation.

In some embodiments, a material of the substrate includes a hydrophilic material.

In some embodiments, after a modification operation is performed on the interior of each of the plurality of culture regions, a hydrophilicity of the interior of each of the plurality of culture regions is greater than a hydrophilicity of the exterior of each of the plurality of culture regions, the modification operation including at least one of a plasma operation, a radiation irradiation operation, or a corona operation.

In some embodiments, a material of the substrate includes a hydrophobic material.

In some embodiments, after a modification operation is performed on the interior of each of the plurality of culture regions, the interior of each of the plurality of culture regions has hydrophilicity, the modification operation including at least one of a plasma operation, a radiation irradiation operation, or a corona operation.

In some embodiments, a material of the substrate includes at least one of glass, quartz, silicon, mica, polystyrene (PS), polymethyl methacrylate (PMMA), polysulfone (PSU), polycarbonate (PC), polypropylene (PP), polyethylene (PE), polyethylene terephthalate glycol (PETG), low-density polyethylene (LDPE), high-density polyethylene (HDPE), polyethylene terephthalate (PET), polyvinylidene fluoride (PVDF), polytetrafluoroethylene (PTFE), polyethylene glycol (PEG), polyethylene oxide (PEO), polypropylene glycol (PPG), polylactic acid (PLA), polyglycolic acid (PGA), polylacticcoglycollic acid (PLGA), polydimethylsiloxane (PDMS), polyvinyl acetate (PVA), copolymers of cycloolefin (COC), copolymers of polypropylene (COP), polymethylpentene (PMP), polystyrene/butadiene copolymer, polystyrene/acrylonitrile copolymer, cellulose acetate, nitrocellulose, hydroxyethyl methylacrylate (HEMA), polyethersulfone, divinyl glycol polymer, or nylon 66.

In some embodiments, the surface of the substrate is modified with a hydrophilic substance or a hydrophilic functional group, wherein the hydrophilic substance includes at least one of collagen, fibronectin, laminin, polylysine, gelatin, hyaluronic acid, chitosan, arginine-glycine-aspartic (RGD) peptides, deoxyribonucleic acid (DNA), lysine, metallic gold, or block polyether, and the hydrophilic functional group includes at least one of a hydroxyl group, a carboxyl group, a carbonyl group, an amino group, a thiol group, a sulfonic acid group, a phosphate group, a quaternary ammonium group, an ether bond, a carboxylic ester group, or an amide group.

In some embodiments, the surface of the substrate is modified with a hydrophobic substance or a hydrophobic functional group, wherein the hydrophobic substance includes albumin, and the hydrophobic functional group includes at least one of an alkyl group containing one or more double bonds, a polyoxypropylene group, a long-chain perfluoroalkyl group, a polysiloxane group, or a hydroxyl group containing aromatic, ester, ether, amine, or amide group.

In some embodiments, the interior of each of the plurality of culture regions is modified with a hydrophilic substance or a hydrophilic functional group.

In some embodiments, the exterior of each of the plurality of culture regions is modified with a hydrophobic substance or a hydrophobic functional group.

In some embodiments, a hydrophilicity of the interior of each of the plurality of culture regions is stronger than a hydrophilicity of the exterior of each of the plurality of culture regions.

In some embodiments, the culture device includes a base plate and a cover plate, the base plate and the cover plate are movable with respect to each other; and the surface of the substrate is an upper surface of the base plate or a lower surface of the cover plate.

In some embodiments, the upper surface of the base plate includes the plurality of culture regions; and the adhesion affinity of the interior of each of the plurality of culture regions towards the culture is greater than an adhesion affinity of the lower surface of the cover plate towards the culture.

In some embodiments, the lower surface of the cover plate includes the plurality of culture regions; and the adhesion affinity of the interior of each of the plurality of culture regions towards the culture is greater than an adhesion affinity of the upper surface of the base plate towards the culture.

In some embodiments, the upper surface of the upper surface of the base plate includes at least two culture regions, and the lower surface of the cover plate also includes at least two culture regions.

In some embodiments, the at least two culture regions on the upper surface of the base plate are staggered with the at least two culture regions on the lower surface of the cover plate.

In some embodiments, a periphery of the base plate is connected to a periphery of the cover plate through an extendable corrugated tube.

In some embodiments, a periphery of the base plate is connected to a periphery of the cover plate through a flexible membrane and a support frame.

In some embodiments, both ends of the support frame are connected to the base plate and the cover plate, respectively, and the support frame undergoes plastic deformation during a transition from a compressed state to an extended state to support the cover plate.

In some embodiments, the base plate or the cover plate is configured with a culture inlet; and the cover plate is configured with a gas inlet and outlet.

In some embodiments, an upper surface of the cover plate is configured with a gripping handle.

In some embodiments, the culture device further comprises a telescoping mechanism, and the telescoping mechanism is configured to drive the base plate and the cover plate to move relative to each other.

In some embodiments, the telescoping mechanism includes a gas control mechanism, and the gas control mechanism is configured to inflate gas between the base plate and the cover plate such that the base plate and the cover plate move away from each other.

In some embodiments, the culture device further includes a side wall, and the side wall is connected to the base plate to form a culture container; and the cover plate are movable with respect to the base plate in the culture container.

In some embodiments, a periphery of the cover plate includes a sealing ring, and the cover plate abuts against the side wall through the sealing ring.

In some embodiments, a roughness of the interior of each of the plurality of culture regions is different from a roughness of the exterior of each of the plurality of culture regions, such that the adhesion affinity of the interior of each of the plurality of culture regions towards the culture is greater than the adhesion affinity of the exterior of each of the plurality of culture regions towards the culture.

In some embodiments, the upper surface of the base plate or the lower surface of the cover plate is hydrophilic, and the roughness of the interior of each of the plurality of culture regions is greater than the roughness of the exterior of each of the plurality of culture regions.

In some embodiments, the upper surface of the base plate or the lower surface of the cover plate is hydrophobic, the roughness of the interior of each of the plurality of culture regions is less than the roughness of the exterior of each of the plurality of culture regions, and a surface of the exterior of each of the plurality of culture regions has an irregular structure.

In some embodiments, a surface of the interior of each of the plurality of culture regions has a regular structure at submicron to nanometer level, such that the adhesion affinity of the interior of each of the plurality of culture regions is greater than the adhesion affinity of the exterior of each of the plurality of culture regions.

Some embodiments of the present disclosure provide a culture method, wherein the culture method use the culture device according to any embodiment of the present disclosure to culture.

In some embodiments, the culture device includes a base plate and a cover plate, the base plate and the cover plate are movable with respect to each other, and the substrate surface is the upper surface of the base plate or the lower surface of the cover plate. The culture method includes: adding a culture between the base plate and the cover plate when the base plate and the cover plate are in a first relative position where the base plate and the cover plate are relatively close; and driving the base plate and the cover plate to move relative to each other to a second relative position where the base plate and the cover plate are relatively far, such that the culture automatically adheres to the plurality of culture regions.

In some embodiments, the culture method further includes: driving the base plate and the cover plate to move relative to each other to simulate a regular motion of blood circulation.

### BRIEF DESCRIPTION OF THE DRAWINGS

The present disclosure is further illustrated in terms of exemplary embodiments. These exemplary embodiments are described in detail according to the drawings. These embodiments are non-limiting exemplary embodiments, in which like reference numerals represent similar structures, wherein:
FIG. 1 is a schematic diagram illustrating a culture device according to some embodiments of the present disclosure;
FIG. 2 is a schematic diagram illustrating a side view of a culture device according to some embodiments of the present disclosure;
FIG. 3 is a schematic diagram illustrating an exploded view of a culture device according to some embodiments of the present disclosure;
FIG. 4 is a schematic diagram illustrating a structure of a culture device in a compressed state according to some embodiments of the present disclosure;
FIG. 5 is a schematic diagram illustrating a structure of a culture device in an extended state according to some embodiments of the present disclosure;
FIG. 6 is a schematic diagram illustrating a perspective view of a culture device in a compressed state according to some embodiments of the present disclosure;
FIG. 7 is a schematic diagram illustrating a perspective view of a culture device in an extended state according to some embodiments of the present disclosure;
FIG. 8 is a schematic diagram illustrating a perspective view of a culture device in a compressed state according to another embodiment of the present disclosure;
FIG. 9 is a schematic diagram illustrating a perspective view of a culture device in an extended state according to another embodiment of the present disclosure;
FIG. 10 is a schematic diagram illustrating structures of a flexible membrane and a support frame according to some embodiments of the present disclosure;
FIG. 11 is a schematic diagram illustrating a regular structure of a surface of an interior of a culture region according to some embodiments of the present disclosure;
FIG. 12 is a schematic diagram of a regular structure of a surface of an interior of a culture region according to another embodiment of the present disclosure; and
FIG. 13 is a schematic diagram of a regular structure of a surface of an interior of a culture region according to yet another embodiment of the present disclosure.

In the drawings, 100 represents a culture device, 110 represents a substrate, 112 represents a base plate, 114 represents a cover plate, 120 represents culture regions, 122 represents micro-nano monomers, 130 represents a non-culture region, 140 represents an etched surface, 150 represents a polished surface, 160 represents an extendable corrugated tube, 172 represents a culture inlet, 174 represents a gas inlet and outlet, 182 represents a first gripping handle, 184 represents a second gripping handle, 190 represents a flexible membrane, and 192 represents a support frame.

### DETAILED DESCRIPTION

In order to provide a clearer and more comprehensive understanding of the purposes, technical solutions, and advantages of the present disclosure, further detailed descriptions of the present disclosure will be provided below, in conjunction with the accompanying drawings and embodiments. It should be understood that the specific embodiments described herein are intended merely for explaining the present disclosure and are not intended to limit the scope of the present disclosure.

Conversely, the present disclosure encompasses any alternate, modified, or equivalent methods and approaches that fall within the essence and scope of the claims. Furthermore, to enhance public comprehension of the present disclosure, specific details are extensively described in the detailed descriptions of the present disclosure below. Those skilled in the art may understand the present disclosure even without these detailed descriptions.

Embodiments of the present disclosure relate to a culture device. The culture device may be used to culture organisms (e.g., cells, bacteria, viruses, etc.) in vitro. Taking cell culture as an example, a culture that can be cultured in the culture device may include a two-dimensional (2D) adherent cell, a suspension fractionated cell, a three-dimensional (3D) cell cluster, an organoid, a tissue-like substance, an ex vivo living tissue, an ex vivo living organ, a non-ex vivo tissue, a non-ex vivo organ, a cell-microcarrier complex, a cell-scaffold complex, or the like, or any combination thereof. The scaffold may include a gel material, a tissue-engineered porous scaffold, etc.

In some embodiments, the culture device may be used for 3D cell culture. The 3D cell culture refers to culturing cells to be cultured in a carrier (or the scaffold) that provides a 3D environment for growth of the cells to be cultured, so that the cells to be cultured are maintained in a growth state in the 3D environment, and ultimately a 3D cell culture product (e.g., an organoid, a tissue-like substance, etc.) is obtained. In some embodiments, a matrix gel (e.g., a temperature-sensitive gel) may be used as the carrier (or the scaffold) for the 3D cell culture. In some embodiments, the cells to be cultured may be mixed in a liquid matrix gel before inoculating the culture, the liquid matrix gel may undergo a phase transition from a liquid state to a solid (or gelled) state under a certain condition, and the solid (or gelled) matrix gel may provide the 3D environment for the cells to be cultured to grow. In some embodiments, the matrix gel may be the temperature-sensitive gel, and the temperature-sensitive gel may undergo the phase transition with temperature changes. The temperature-sensitive gel may include poly N-isopropylacrylamide (PNIPAM), block copolymer of poly N-isopropylacrylamide and polyethylene glycol (PNIPAM-PEG), polyethylene glycol (PEG), block copolymer of polylactic acid and poly lactic-co-glycolic acid (PLA-PLGA), triblock copolymer of poly(lactic-co-glycolic acid)-poly(ethylene glycol)-poly(lactic-co-glycolic acid) (PLGA-PEG-PLGA), or the like, or any combination thereof. In some embodiments, the culture may be understood as a matrix gel mixed with the cells to be cultured. In some alternative embodiments, the culture device may be used for 2D cell culture. In some embodiments, after a liquid culture transforms into the solid (or gelled) state, a culture medium may be added to the culture device. The culture medium may be a nutrient solution for the growth and reproduction of the culture, which may be prepared by combining different nutrients.

In some embodiments, the culture device may include one or more culture chambers. A culture chamber refers to a device or structure that provides space for the survival, growth, and reproduction of the cells to be cultured. In some embodiments, the one or more culture chambers may include a culture dish, a culture bottle, a culture plate, a culture column, etc. A substrate may be understood as a portion (e.g., a bottom wall of the culture chamber) of the culture chamber that is in contact with the culture.

During a culture process (taking the cell culture as an example), a crucial operation is the inoculation of the culture, which refers to adding the culture into the culture device. In some embodiments, the inoculation of the cell culture may be performed manually. For example, an operator utilizes a pipette gun to inoculate the cultures one by one (or row by row using a pin header) onto the substrate of the culture device. The one by one manual inoculation is inefficient, slow, which may affect the inoculated culture, thereby affecting the quality of the cell culture product. For example, when the culture is the matrix gel mixed with the cells to be cultured, due to a narrow temperature range of the phase transition of the matrix gel (e.g., the temperature-sensitive gel liquefies at 4 degrees centigrade (°C), and gradually solidifies when the temperature exceeds 10 °C), if the temperature is not properly controlled, the culture may solidify before the inoculation is completed, which not only reduces the efficiency of the cell culture, but also results in that the cell culture product does not satisfy culture requirement(s).

Some embodiments of the present disclosure provide a culture device. By designing alternately varying patterned arrays on a surface of a substrate of the culture device, a liquid culture (e.g., the matrix gel mixed with the cells to be cultured) can automatically stay on culture region(s) with a relatively large adhesion affinity (e.g., culture region(s) with a relatively large roughness, a relatively large hydrophilicity, a relatively weak hydrophobicity, having a regular structure at submicron to nanometer level, etc.) when the culture is inoculated. Therefore, multiple dispensing can be automatically formed during a single liquid addition. Using the culture device, the speed of the inoculation can be increased, the efficiency of the cell culture can be improved, and the quality of the cell culture product can be improved.

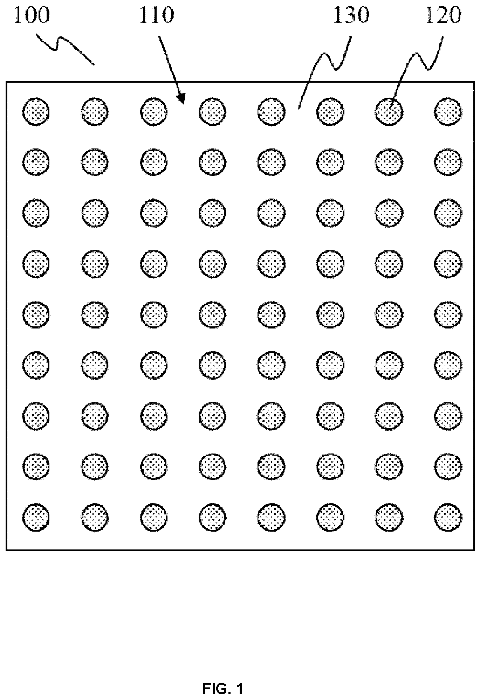

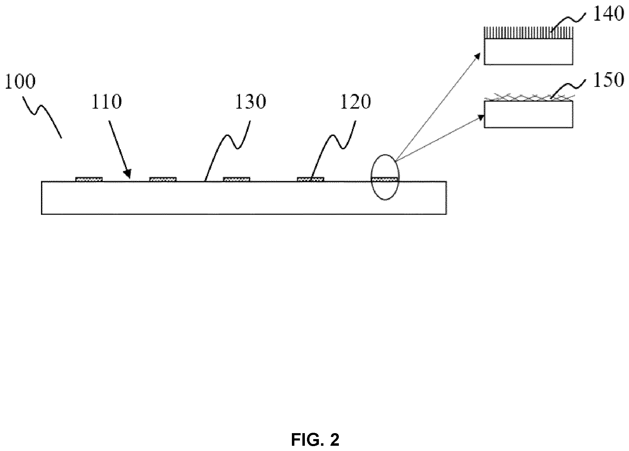

FIG. 1 is a schematic diagram illustrating a culture device according to some embodiments of the present disclosure. FIG. 2 is a schematic diagram illustrating a side view of a culture device according to some embodiments of the present disclosure. Referring to FIGs. 1 and 2, a culture device 100 disclosed by the present disclosure may be described below. It should be noted that, the following descriptions are merely for illustration purposes, and are not intended to limit the scope of the present disclosure.

As shown in FIGs. 1 and 2, the culture device 100 may include a substrate 110, and a surface of the substrate 110 may include a plurality of culture regions 120. The plurality of culture regions 120 may be used to adhere a culture (e.g., a matrix gel mixed with cells to be cultured). In some embodiments, the plurality of culture regions 120 may have a regular shape. For example, the shape of the plurality of culture regions 120 may include a circle, a square, a triangle, an oval, etc. In some embodiments, the plurality of culture regions 120 may also have an irregular shape. In some embodiments, the shape of the plurality of culture regions 120 may be the circle, and the circle can increase a contact area between the culture and a culture medium (e.g., a culture fluid) after inoculation is completed, thereby increasing a nutrient absorption rate.

In some embodiments, shapes and sizes of the plurality of culture regions 120 may be the same, allowing for rapid and batched inoculation of cultures with a same or substantially same volume during the cell culture. In practice, the volume of the added culture may be determined based on a desired size of the dispensing and a count of the plurality of culture regions 120 in the culture device. After a liquid-like culture is added to the culture device 100, the culture may be adhered to each culture region 120 in a substantially uniform manner by shaking and tilting the culture device. After the liquid-like culture turns into the solid (or gelled) state, the culture medium may be added to the culture device. The culture medium may be a nutrient solution for the growth and reproduction of the culture, which may be prepared by combining different nutrients.

In some embodiments, the plurality of culture regions 120 may be arranged randomly or according to a certain rule. For example, the plurality of culture regions 120 may be arranged at equal distances from each other. In some embodiments, sizes and spacings of the plurality of culture regions 120 may be limited to ensure uniform and rapid absorption of the culture medium by the cells to be cultured. In some embodiments, a diameter of a circular culture region (i.e., when the culture region 120 is circular) may be set in a range from 0.1 millimeters to 50 millimeters. In some embodiments, the diameter of the circular culture region may be set in a range from 0.2 millimeters and 10 millimeters. In some embodiments, the diameter of each circular culture region may be set in a range from 1 millimeter and 5 millimeters. In some embodiments, a spacing between any two culture regions may be greater than the diameter of the culture region. In some embodiments, the spacing between any two culture regions may be greater than 1.5 times the diameter of the culture region.

In some embodiments, a roughness of an interior of each culture region may be different from a roughness of an exterior of each culture region, such that an adhesion affinity of the interior of each culture region to the culture is greater than an adhesion affinity of the exterior of each culture region to the culture, thereby improving the adhesion of the culture to the interior of each culture region 120.

In some embodiments, a surface of the substrate 110 may be hydrophilic, and the roughness of the interior of each culture region 120 may be greater than the roughness of the exterior (e.g., a non-culture region 130) of each culture region 120. In some embodiments, the hydrophilicity of the surface of the substrate 110 can be achieved using the following manners: by using a hydrophilic material as a material of the substrate 110, by modifying part or all of the surface of the substrate 110, modifying part or all of the surface of the substrate 110 with a hydrophilic substance or a hydrophilic functional group (hydrophilic modification), or the like, or any combination thereof. By making the roughness of the interior of each culture region 120 be greater than the roughness of the exterior of each culture region 120, the adhesion affinity of the interior of each culture region to the culture may be greater than the adhesion affinity of the exterior of each culture region to the culture, thereby improving the adhesion of the culture to the interior of each culture region 120. In addition, during the culture process, culture droplets adhered to the interior of each culture region 120 may have a relatively flattened shape. After the flattened droplets are solidified into the gelled state, the flattened droplets may contribute to improving the penetration of the external culture medium (e.g., the culture fluid) into the interior.

In some embodiments, the roughness of the interior of each culture region 120 being greater than the roughness of the exterior of each culture region 120 may be achieved through various manners. In some embodiments, a polishing operation may be performed on the interior of each culture region 120, such that the roughness of the interior of each culture region 120 is greater than the roughness of the exterior of each culture region 120. For example, the interior of each culture region may be polished using sandpaper, pumice, etc., with a specific roughness, such that the roughness of the interior of each culture region 120 is greater than the roughness of the exterior of each culture region 120. As shown in FIG. 2, a polished interior of a culture region 120 may be illustrated as a polished surface 150. The polished surface 150 may be an irregular rough surface (or an irregular structure). Polishing is an advantageous way to make a difference between the roughnesses of the interior and exterior of each culture region, since it is simple, efficient, low-cost, etc. In some alternative embodiments, the exterior of each culture region may be polished (or buffed), such that the roughness of the interior of each culture region is greater than the roughness of the exterior of each culture region. In some embodiments, an etching operation may be performed on the interior of each culture region, such that the roughness of the interior of each culture region is greater than the roughness of the exterior of each culture region. In some embodiments, the etching operation may include a soft etching operation, a laser etching operation, a plasma etching operation, an electron beam etching operation, a chemical etching operation, or the like, or any combination thereof. As shown in FIG. 2, an etched interior of a culture region 120 may be illustrated as an etched surface 140. The etched surface 140 may include a regular rough surface. Etching is an advantageous way to make a difference between the roughnesses of the interior and exterior of each culture region, since it has excellent roughness control, high operating efficiency, stability, uniformity among the plurality of culture regions, etc.

In some embodiments, the surface of the substrate 110 may be hydrophobic, the roughness of the interior of each culture region 120 may be less than the roughness of the exterior of each culture region 120, and a surface of the exterior of each culture region may have an irregular structure. In some embodiments, the hydrophobicity of the surface of the substrate 110 can be achieved using the following manners: by using a hydrophobic material as a material of the substrate 110, by modifying part or all of the surface of the substrate 110 with a hydrophobic substance or a hydrophobic functional group (hydrophobic modification), etc. By making the roughness of the interior of each culture region 120 be greater than the roughness of the exterior of each culture region 120 on the hydrophobic surface of the substrate 110, and making the surface of the exterior of each culture region 120 have the irregular structure, the adhesion affinity of the interior of each culture region to the culture may be greater than the adhesion affinity of the exterior of each culture region to the culture, thereby improving the adhesion of the culture to the interior of each culture region 120. In addition, during the culture process, the culture droplets adhered to the interior of each culture region 120 may have an approximately spherical shape. After the spherical shaped droplets are solidified into the gelled state, the spherical shaped droplets may contribute to providing a more 3D growth space for the culture, thereby facilitating the culture of a complex structure (e.g., an organoid). In some embodiments, the exterior of each culture region may be polished, such that the roughness of the interior of each culture region is less than the roughness of the exterior of each culture region. In some alternative embodiments, the interior of each culture region may be polished (or buffed), such that the roughness of the interior of each culture region is less than the roughness of the exterior of each culture region.

In some embodiments, the surface of the interior of each culture region may have a regular structure at submicron to nanometer level, such that the adhesion affinity of the interior of each culture region to the culture is greater than the adhesion affinity of the exterior of each culture region to the culture, thereby improving the adhesion of the culture to the interior of each culture region 120. In some embodiments, the regular structure at submicron to nanometer level may include a plurality of micro-nano monomers arranged in an array. A micro-nano monomer may include a micro-nano pillar, a micro-nano tube, a micro-nano cone, a micro-nano wall, or the like, or any combination thereof. In some embodiments, the plurality of micro-nano monomers may be arranged in a circular array, a rectangular array, etc. In some embodiments, the spacing between any two of the plurality of micro-nano monomers may be the same or different. In some embodiments, when the surface of the interior of each culture region has the regular structure at submicron to nanometer level, the surface of the exterior of each culture region may have an irregular structure (i.e., do not have the regular structure).

Some embodiments of the present disclosure provide a culture device. By designing alternately varying patterned arrays on an upper surface of a base plate or a lower surface of a cover plate of the culture device, a liquid culture (e.g., a matrix gel mixed with cells to be cultured) can automatically stay on culture region(s) with a relatively large adhesion affinity (e.g., culture region(s) with a relatively large roughness, a relatively large hydrophilicity, a relatively weak hydrophobicity, having a regular structure at submicron to nanometer level, etc.) when the culture is inoculated. Therefore, multiple dispensing can be automatically formed during a single liquid addition. Using the culture device, the speed of the inoculation can be increased, the efficiency of the cell culture can be improved, and the quality of the cell culture product can be improved. In some embodiments, a substrate of the culture device may be the base plate or the cover plate. A surface of the substrate may be the upper surface of the base plate or the lower surface of the cover plate.

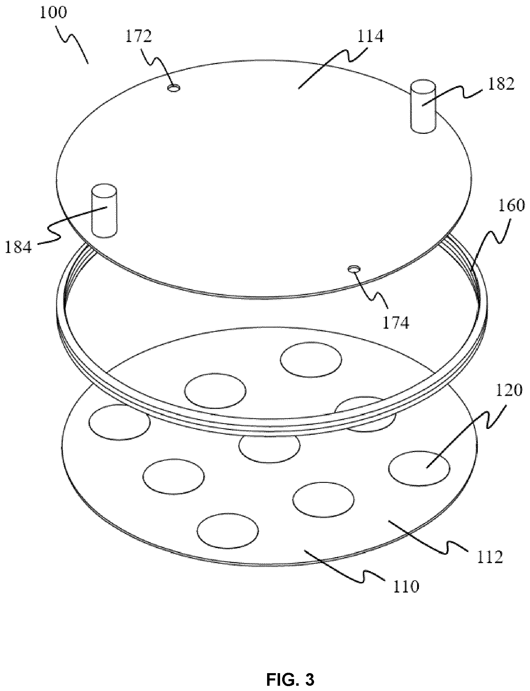

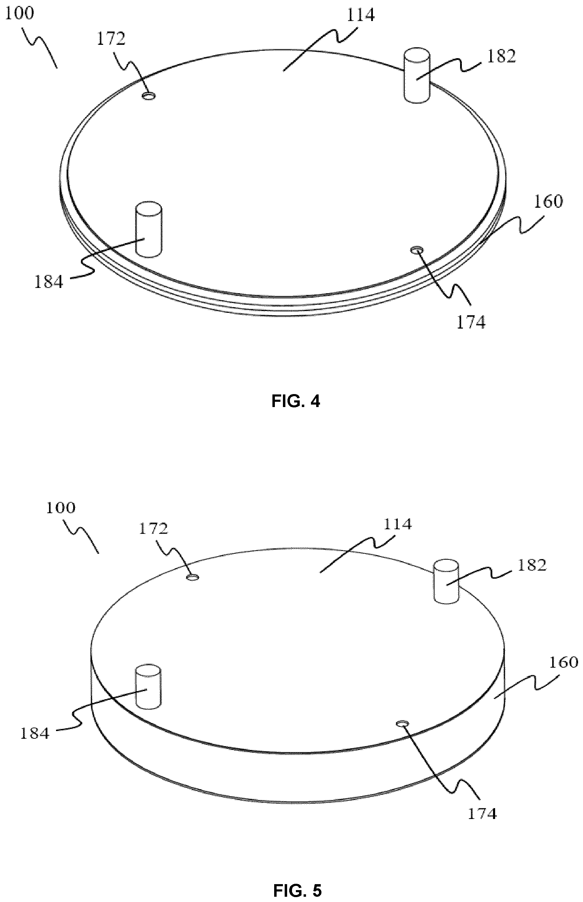

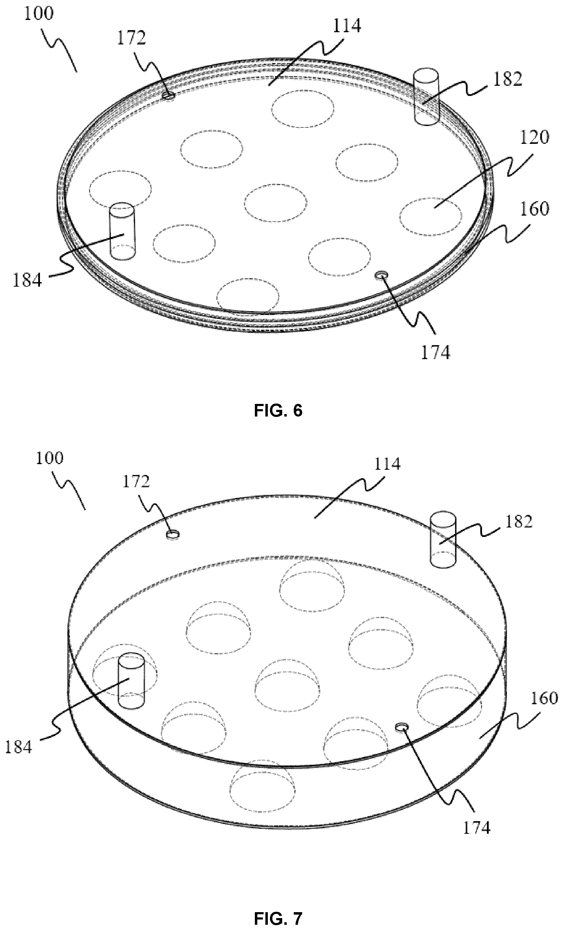

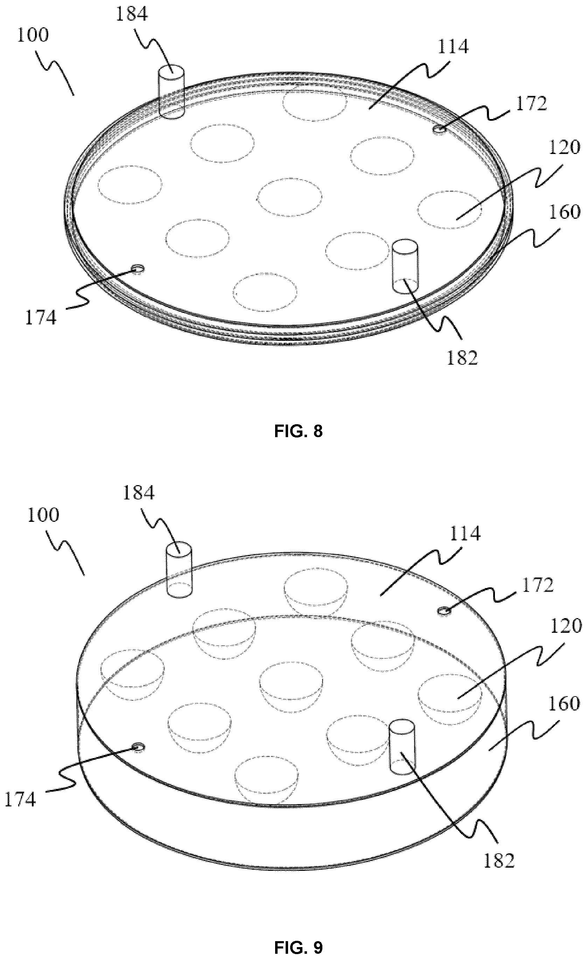

FIG. 3 is a schematic diagram illustrating an exploded view of a culture device according to some embodiments of the present disclosure. FIG. 4 is a schematic diagram illustrating a structure of a culture device in a compressed state according to some embodiments of the present disclosure. FIG. 5 is a schematic diagram illustrating a structure of a culture device in an extended state according to some embodiments of the present disclosure. FIG. 6 is a schematic diagram illustrating a perspective view of a culture device in a compressed state according to some embodiments of the present disclosure. FIG. 7 is a schematic diagram illustrating a perspective view of a culture device in an extended state according to some embodiments of the present disclosure. FIG. 8 is a schematic diagram illustrating a perspective view of a culture device in a compressed state according to another embodiment of the present disclosure. FIG. 9 is a schematic diagram illustrating a perspective view of a culture device in an extended state according to another embodiment of the present disclosure. Referring to FIGs. 3-9, a culture device 100 disclosed by the present disclosure may be described below. It should be noted that, the following descriptions are merely for illustration purposes, and are not intended to limit the scope of the present disclosure.

As shown in FIGs. 3-9, the culture device 100 may include a base plate 112 and a cover plate 114, and the base plate 112 and the cover plate 114 may be movable with respect to each other. An upper surface of the base plate 112 or a lower surface of the cover plate 114 may include a plurality of culture regions 120. The plurality of culture regions 120 may be used to adhere the culture (e.g., a matrix gel mixed with cells to be cultured), and an adhesion affinity of the interior of each culture region 120 to the culture may be greater than an adhesion affinity of the upper surface of the base plate 112 to the culture. In some embodiments, the plurality of culture regions 120 may have a regular shape. For example, the shape of the plurality of culture regions 120 may include a circle, a square, a triangle, an oval, etc. In some embodiments, the plurality of culture regions 120 may also have an irregular shape. In some embodiments, the shape of the plurality of culture regions 120 may be the circle, and the circle can increase a contact area between the culture and a culture medium (e.g., a culture fluid) after inoculation is completed, thereby increasing a nutrient absorption rate. In some embodiments, shapes and sizes of the plurality of culture regions 120 may be the same, allowing for rapid and batched inoculation of a plurality of cultures with a same or substantially same volume during the cell culture.

In some embodiments, the plurality of culture regions 120 may be arranged randomly or according to a certain rule. For example, the plurality of culture regions 120 may be arranged at equal distances from each other (e.g., arranged in an array). In some embodiments, sizes and spacings of the plurality of culture regions 120 may be limited to ensure uniform and rapid absorption of the culture medium by the cells to be cultured. In some embodiments, a diameter of a circular culture region (i.e., when the culture region 120 is circular) may be set in a range from 0.1 millimeters to 50 millimeters. In some embodiments, the diameter of the circular culture region may be set in a range from 0.2 millimeters and 10 millimeters. In some embodiments, the diameter of each circular culture region may be set in a range from 1 millimeter and 5 millimeters. In some embodiments, a spacing between any two culture regions may be greater than the diameter of the culture region. In some embodiments, the spacing between any two culture regions may be greater than 1.5 times the diameter of the culture region.

In some embodiments, as shown in FIGs. 3-5, the upper surface of the base plate 112 may include a plurality of culture regions 120. The adhesion affinity of the interior of each culture region 120 to the culture may be greater than the adhesion affinity of the exterior of each culture region 120 to the culture, and be also greater than an adhesion affinity of the lower surface of the cover plate 114 to the culture. Thereby, the culture may be adhered to the plurality of culture regions 120 on the upper surface of the base plate 112.

In some embodiments, as shown in FIGs. 6-9, the lower surface of the cover plate 114 may include the plurality of culture regions 120. The adhesion affinity of the interior of each culture region 120 to the culture may be greater than the adhesion affinity of the exterior of each culture region 120 to the culture, and also greater than the adhesion affinity of the upper surface of the base plate 112 to the culture. Therefore, the culture may be adhered to the plurality of culture regions 120 on the lower surface of the cover plate 114. By disposing the plurality of culture regions on the lower surface of the cover plate 114, culture droplets may be hung upside down under the cover plate 114. Due to the gravity, the cells to be cultured in the culture may tend to grow downward in the process of extension, thereby reducing wall-adherent cell growth. The approach of setting the plurality of culture regions 120 on the lower surface of the cover plate 114 may be used for organoid culture, the culture of 3D cell clusters, etc. Under the effect of gravity, suspended cells within the droplets may automatically gather at bottoms of the droplets, thereby forming the 3D cell clusters. In some embodiments, the approach may be an alternative to a suspended drip plate and a U-shaped bottom/spherical bottom culture plate.

In some embodiments, the upper surface of the base plate 112 may include a plurality of culture regions 120, and the lower surface of the cover plate 114 may also include a plurality of culture regions 120. By setting the plurality of culture regions 120 on both the upper surface of the base plate 112 and the lower surface of the cover plate 114, culture droplets that are both upright and hanging upside down may be formed simultaneously in one liquid addition. In some embodiments, the plurality of culture regions on the upper surface of the base plate and the plurality of culture regions on the lower surface of the cover plate may be disposed opposite to each other. In some embodiments, the plurality of culture regions on the upper surface of the base plate may be staggered with the plurality of culture regions on the lower surface of the cover plate. The staggered arrangement of the plurality of culture regions 120 on the upper surface of the base plate 112 and the plurality of culture regions 120 on the lower surface of the cover plate 114 may be easy to observe during the culture process, and effectively improve the utilization efficiency of the culture device 100.

In some embodiments, as shown in FIGs. 3-9, a periphery of the base plate may be connected to a periphery of the cover plate through an extendable corrugated tube 160. In some embodiments, the extendable corrugated tube 160 may be made of materials, such as polypropylene (PP), polyethylene (PE), polyethylene terephthalate (PET), etc. In some embodiments, a processing technique for the extendable corrugated tube 160 may include a mechanical processing technique, a cold rolling technique, an oblique rolling technique, a continuous extrusion technique, a hot rolling technique, a 3D printing technique, or the like, or any combination thereof. By using the extendable corrugated tube 160 to connect the base plate 112 and the cover plate 114, a relative movement between the base plate 112 and the cover plate 114 can be convenient and stable. The extendable corrugated tube 160 may also ensure the sealing of a chamber formed between the base plate 112 and the cover plate 114. The sealing of the chamber formed between the base plate 112 and the cover plate 114 can prevent the liquid culture within the chamber from overflowing. In some embodiments, the extendable corrugated tube 160 may be connected to the base plate 112 and/or the cover plate 114 through manners, such as an adhesive connection, a welding (e.g., laser welding, ultrasonic welding, heat welding) connection, a clamping connection, etc. In some embodiments, the extendable corrugated tube 160 may be integrally molded and formed with the base plate 112 and/or the cover plate 114 through operations, such as an injection molding operation, a mold molding operation, a 3D printing operation, etc. In some embodiments shown in FIGs. 3-9, FIGs 3, 4, 6, and 8 illustrate the extendable corrugated tube 160 in a compressed state, and FIGs. 5, 7, and 9 illustrate the extendable corrugated tube 160 in an extended state.

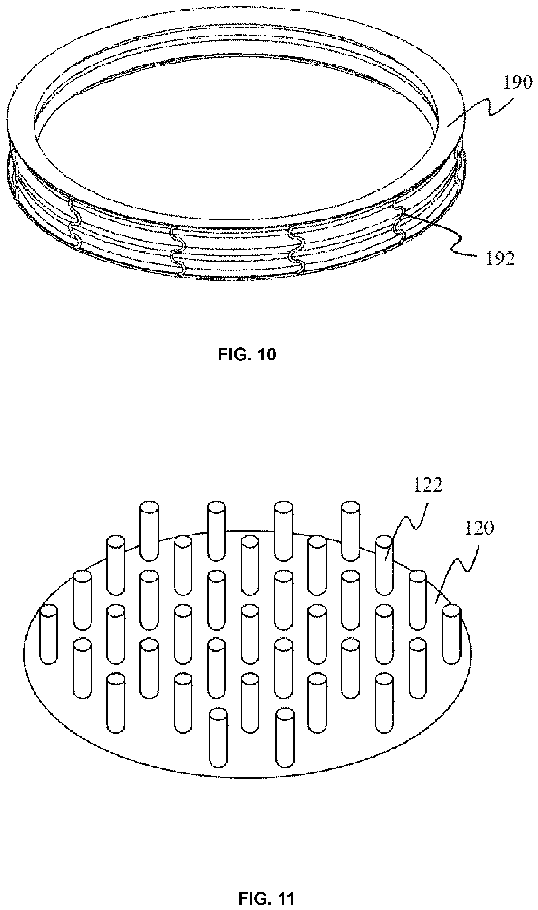

In some embodiments, the periphery of the base plate 112 and the periphery of the cover plate 114 may be connected through a flexible membrane 190 and a support frame 192. FIG. 10 is a schematic diagram illustrating structures of a flexible membrane and a support frame according to some embodiments of the present disclosure. As shown in FIG. 10, the support frame 192 may be disposed outside the flexible membrane 190. In some embodiments, both ends of the support frame 192 may be connected to the base plate 112 and the cover plate 114, respectively. The support frame 192 may undergo plastic deformation during a transition from the compressed state (not shown in the figure) to the extended state (as shown in FIG.10) to support the cover plate 114. In some embodiments, the support frame 192 may be made of a metallic material (e.g., 316 stainless steels, etc.), a polymeric material (e.g., polylactic acid (PLA), etc.), etc. In some embodiments, as shown in FIG. 10, a plurality of support frames 192 may be evenly disposed around the peripheries of the base plate 112 and the cover plate 114. In some embodiments, the support frame 192 and the flexible membrane 190 may be fixedly connected through manners, such as a gluing connection. In some embodiments, the support frame 192 and the flexible membrane 190 may not be fixedly connected. In some embodiments, the support frame 192 and the flexible membrane 190 may be fixed to the periphery of the base plate 112 and the periphery of the cover plate 114 (e.g., through a welding connection, a gluing connection, etc.), respectively. By disposing the flexible membrane 190 and the support frame 192, the relative movement (e.g., transitioning from the compressed state to the extended state) between the base plate 112 and the cover plate 114 can be convenient and stable. The flexible membrane 190 may ensure the sealing of the chamber formed between the base plate 112 and the cover plate 114. By using a plastic material to prepare the support frame 192, the culture device 100 can maintain its shape after the culture device 100 is transitioned into the stretched state, thereby facilitating the use of the culture device 100.

In some embodiments, the base plate 112 or the cover plate 114 may be configured with a culture inlet 172. The cover plate 114 may be configured with a gas inlet and outlet 174. In some embodiments, as shown in FIGs. 3-7, both the culture inlet 172 and the gas inlet and outlet 174 may be disposed on the cover plate 114. The culture inlet 172 may be used to add the culture into the chamber formed between the base plate 112 and the cover plate 114. The gas inlet and outlet 174 may be used for the gas to enter and exit the chamber. In some embodiments, a count of the culture inlet 172 may be one or more. In some embodiments, a count of the gas inlet and outlet 174 may be one or more. In some embodiments, as shown in FIGs. 3-7, the culture inlet 172 and the gas inlet and outlet 174 may be disposed at both radial ends of the cover plate 114, respectively, such that the gas in the chamber may be facilitated to be discharged from the gas inlet and outlet 174 when the culture is added to the chamber from the culture inlet 172. In some embodiments, the culture inlet 172 and/or the gas inlet and outlet 174 may be formed through operation(s), such as a laser cutting operation, a thermal cutting operation, a water cutting operation, etc. In some embodiments, the culture inlet 172 and/or the gas inlet and outlet 174 may be formed during the manufacturing process (e.g., a 3D printing operation, an injection molding operation, a mold molding operation, etc.) of the base plate 112 or the cover plate 114.

In some embodiments, an upper surface of the cover plate 114 may be configured with a gripping handle. The gripping handle may be used to extend and/or compress the cover plate 114, thereby causing the cover plate 114 to move with respect to the base plate 112. In some embodiments, the gas inlet and outlet 174 may be opened when the gripping handle extends and/or compresses the cover plate 114. In some embodiments, the gripping handle may be used to be held by a user. In some embodiments, the gripping handle may be used to be gripped by a device (e.g., a tweezer, etc.). In some embodiments, the gripping handle may be used to be connected to a telescoping mechanism. In some embodiments, as shown in FIGs. 3-7, the gripping handle may include a first gripping handle 182 and a second gripping handle 184. The first gripping handle 182 and the second gripping handle 184 may be disposed at both radial ends of the cover plate 114, respectively, such that the cover plate 114 may be subjected to a more uniform force and move more stably when the cover plate 114 is extended and/or compressed by the gripping handle. In some embodiments, a count of the gripping handle may be only one. For example, the single gripping handle may be disposed at a center of the upper surface of the cover plate 114. In some embodiments, the gripping handle may have a shape including a cylinder, a rounded platform shape, a prismatic shape, a handle shape, or the like, or any combination thereof. In some embodiments, a lower surface or the periphery of the base plate 112 may also be configured with a gripping handle. In some embodiments, the gripping handle may be disposed on the base plate 112 and/or the cover plate 114 through a laser welding connection, an ultrasonic welding connection, a thermal welding connection, a gluing connection, etc. In some embodiments, the gripping handle may be integrally molded during the manufacturing process (e.g., the 3D printing operation, the injection molding operation, the mold molding operation, etc.) of the base plate 112 and/or the cover plate 114.

In some embodiments, the culture device further includes the telescoping mechanism (not shown in the figure). The telescoping mechanism may be configured to drive the base plate 112 and the cover plate 114 to move relative to each other.

In some embodiments, the telescoping mechanism may include a gas control mechanism. In some embodiments, the gas control mechanism may be configured to inflate gas between the base plate 112 and the cover plate 114 such that the base plate 112 and the cover plate 114 move away from each other (i.e., the culture device 100 is extended). In some embodiments, the gas control mechanism may be configured to absorb gas from between the base plate 112 and the cover plate 114 such that the base plate 112 and the cover plate 114 move closer to each other (i.e., the culture device 100 is compressed). In some embodiments, the gas control mechanism may include a gas source and a gas control valve. The gas source may be connected to the gas control mechanism through a gas delivery pipe. The gas control valve may be configured to control the gas source to inflate or absorb the gas. By using the gas control mechanism as the telescoping mechanism, the movement between the base plate 112 and the cover plate 114 can be controlled more conveniently and stably.

In some embodiments, the telescoping mechanism may further include other mechanisms. For example, the telescoping mechanism may include a magnetic expansion rod, and one end of the expansion rod may be connected to the cover plate 114 to drive the cover plate 114 to move relative to the base plate 112. As another example, the telescoping mechanism may include a motor-driven screw nut mechanism, a rack and pinion mechanism, a crank slider mechanism, a cam mechanism, etc.

In some embodiments, the culture device 100 may include a side wall (not shown in the figure). The side wall may be connected to the base plate to form a culture container, and the cover plate 114 may move with respect to the base plate 112 within the culture container. In some embodiments, the side wall and the base plate 112 may be fixedly connected to each other through a gluing connection, a welding connection, a clamping connection, etc. In some embodiments, the side wall and the base plate 112 may be integrally molded (e.g., a culture dish). In some embodiments, the periphery of the cover plate 114 may include a sealing ring, and the cover plate 114 may abut against the side wall through the sealing ring. By disposing the sealing ring on the periphery of the cover plate 114, the sealing of the chamber (e.g., the culture container) formed between the base plate 112 and the cover plate 114 can be effectively ensured. In some embodiments, the culture inlet 172 and/or the gas inlet and outlet 174 may be disposed on the side wall.

In some embodiments, the roughness of the interior of each culture region 120 may be different from the roughness of the exterior of each culture region 120, such that the adhesion affinity of the interior of each culture region to the culture is greater than the adhesion affinity of the exterior of each culture region to the culture, thereby improving the adhesion of the culture to the interior of each culture region 120.

In some embodiments, a surface (e.g., the upper surface of the base plate 112 or the lower surface of the cover plate 114) configured with the plurality of culture regions 120 may be hydrophilic, and the roughness of the interior of each culture region 120 may be greater than the roughness of the exterior of each culture region 120. In some embodiments, the base plate 112 or the cover plate 114 configured with the plurality of culture regions 120 may be referred to as the substrate, and the surface configured with the plurality of culture regions 120 may be referred to as the surface of the substrate 110. In some embodiments, the hydrophilicity of the surface of the substrate 110 can be achieved using the following manners: by using a hydrophilic material as a material of the substrate 110, by modifying part or all of the surface of the substrate 110, by modifying part or all of the surface of the substrate 110 with a hydrophilic substance or a hydrophilic functional group (hydrophilic modification), or the like, or any combination thereof. By making the roughness of the interior of each culture region 120 be greater than the roughness of the exterior of each culture region 120, the adhesion affinity of the interior of each culture region to the culture may be greater than the adhesion affinity of the exterior of each culture region to the culture, thereby improving the adhesion of the culture to the interior of each culture region 120. In some embodiments, the roughness of the interior of each culture region 120 being greater than the roughness of the exterior of each culture region 120 may be achieved through various manners. In some embodiments, a polishing operation may be performed on the interior of each culture region 120, such that the roughness of the interior of each culture region 120 is greater than the roughness of the exterior of each culture region 120. For example, the interior of each culture region may be polished using sandpaper, pumice, etc., with a specific roughness, such that the roughness of the interior of each culture region 120 is greater than the roughness of the exterior of each culture region 120. A polished surface may be an irregular rough surface (or an irregular structure). Polishing is an advantageous way to make a difference between the roughnesses of the interior and exterior of each culture region, since it is simple, efficient, low-cost, etc. In some embodiments, the exterior of each culture region may be polished (or buffed), such that the roughness of the interior of each culture region is greater than the roughness of the exterior of each culture region. In some embodiments, an etching operation may be performed on the interior of each culture region, such that the roughness of the interior of each culture region is greater than the roughness of the exterior of each culture region. In some embodiments, the etching operation may include a soft etching operation, a laser etching operation, a plasma etching operation, an electron beam etching operation, a chemical etching operation, or the like, or any combination thereof. An etched surface may include a regular rough surface. In some embodiments, the etched surface may include a plurality of micrometer or even nanoscale columns. Etching is an advantageous way to make a difference between the roughnesses of the interior and exterior of each culture region, since it has excellent roughness control, high operating efficiency, stability, uniformity among the plurality of culture regions, etc.

In some embodiments, a surface (e.g., the upper surface of the base plate 112 or the lower surface of the cover plate 114) configured with the plurality of culture regions 120 may be hydrophobic, and the roughness of the interior of each culture region 120 may be greater than the roughness of the exterior of each culture region 120, and the surface of the exterior of each culture region may have an irregular structure. In some embodiments, the base plate 112 or the cover plate 114 configured with the plurality of culture regions 120 may be referred to as the substrate, and the surface configured with the plurality of culture regions 120 may be referred to as the surface of the substrate 110. In some embodiments, the hydrophobicity of the surface of the substrate 110 can be achieved using the following manners: by using a hydrophobic material as a material of the substrate 110, by modifying part or all of the surface of the substrate 110 with a hydrophobic substance or a hydrophobic functional group (hydrophobic modification), etc. By making the roughness of the interior of each culture region 120 be greater than the roughness of the exterior of each culture region 120 on the hydrophobic surface of the substrate 110, and making the surface of the exterior of each culture region 120 have the irregular structure, the adhesion affinity of the interior of each culture region to the culture may be greater than the adhesion affinity of the exterior of each culture region to the culture, thereby improving the adhesion of the culture to the interior of each culture region 120. In some embodiments, the exterior of each culture region may be polished, such that the roughness of the interior of each culture region is less than the roughness of the exterior of each culture region. In some alternative embodiments, the interior of each culture region may be polished (or buffed), such that the roughness of the interior of each culture region is less than the roughness of the exterior of each culture region.

In some embodiments, the surface of the interior of each culture region may have a regular structure at submicron to nanometer level, such that the adhesion affinity of the interior of each culture region to the culture is greater than the adhesion affinity of the exterior of each culture region to the culture, thereby improving the adhesion of the culture to the interior of each culture region 120. In some embodiments, the regular structure at submicron to nanometer level may include a plurality of micro-nano monomers arranged in an array. The micro-nano monomer may include a micro-nano pillar, a micro-nano tube, a micro-nano cone, a micro-nano wall, or the like, or any combination thereof. In some embodiments, the plurality of micro-nano monomers may be arranged in a circular array, a rectangular array, etc. In some embodiments, the spacing between any two of the plurality of micro-nano monomers may be the same or different. In some embodiments, when the surface of the interior of each culture region has the regular structure at submicron to nanometer level, the surface of the exterior of each culture region may have an irregular structure (i.e., do not have the regular structure).

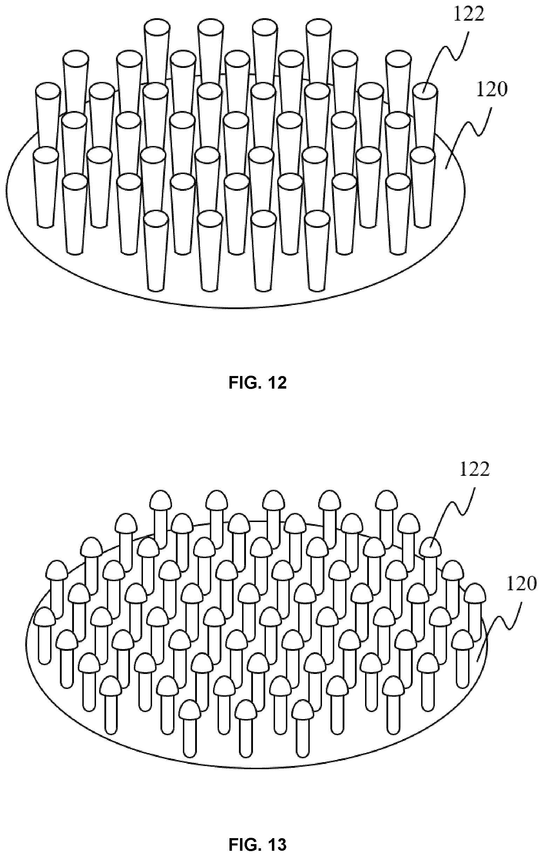

FIG. 11 is a schematic diagram illustrating a regular structure of a surface of an interior of a culture region according to some embodiments of the present disclosure. FIG. 12 is a schematic diagram of a regular structure of a surface of an interior of a culture region according to another embodiment of the present disclosure. FIG. 13 is a schematic diagram of a regular structure of a surface of an interior of a culture region according to yet another embodiment of the present disclosure. As shown in FIGs. 11-13, the surface of the interior of the culture region 120 may have a regular structure at submicron to nanometer level, and the regular structure at submicron to nanometer level may include a plurality of micro-nano monomers 122 arranged in an array. As shown in FIG. 11, the plurality of micro-nano monomers 122 may include micro-nano pillars. As shown in FIG. 12, the plurality of micro-nano monomers 122 may include micro-nano cones. In some embodiments, as shown in FIG. 13, the plurality of micro-nano monomers 122 may include micro-nano pillars, and a head of each of the plurality of micro-nano monomers 122 may have a mushroom-like shape. By disposing the heads of the plurality of micro-nano monomers in the mushroom-like shape, the adhesion affinity of the regular structure at submicron to nanometer level to the culture can be improved. In some embodiments, the heads of the plurality of micro-nano monomer 122 may also have other shapes (e.g., a spherical shape, a planar shape, a wedge shape, etc.).

In some embodiments, a diameter or a maximum width of each of the plurality of the micro-nano monomers may be less than 1 micron. In some embodiments, the diameter or the maximum width of each of the plurality of the micro-nano monomers may be within a range from 1 to 500 nanometers. In some embodiments, the diameter or the maximum width of each of the plurality of the micro-nano monomers may be within a range from 50 to 200 nanometers. In some alternative embodiments, the diameter or the maximum width of each of the plurality of the micro-nano monomers may be greater than or equal to 1 micron. For example, the diameter or the maximum width of each of the plurality of the micro-nano monomers may be within a range from 1 to 100 microns. In some embodiments, a height of each of the plurality of the micro-nano monomers may be less than 5 microns. In some embodiments, the height of each of the plurality of the micro-nano monomers may be within a range from 5 to 1000 nanometers. In some embodiments, the height of each of the plurality of the micro-nano monomers may be within a range from 200 to 500 nanometers. In some embodiments, a ratio of the height to the diameter or the maximum width of each of the plurality of the micro-nano monomers may be within a range from 2:1 to 10:1. In some embodiments, a spacing between any two of the plurality of the micro-nano monomers may be greater than the diameter or the maximum width of each of the plurality of the micro-nano monomers. In some embodiments, the spacing between any two of the plurality of the micro-nano monomers may be greater than 1.5 times the diameter or the maximum width of each of the plurality of the micro-nano monomers. In some embodiments, the spacing between any two of the plurality of the micro-nano monomers may be 1-3 times the diameter or the maximum width of each of the plurality of the micro-nano monomers.

In some embodiments, an etching operation may be performed on the interior of each culture region, such that the surface of the interior of each culture region has the regular structure at submicron to nanometers level. In some embodiments, the etching operation may include a soft etching operation, a laser etching operation, a plasma etching operation, an electron beam etching operation, a chemical etching operation, or the like, or any combination thereof. In some embodiments, a processed regular structure at submicron to nanometers level may be adhered to the surface of the substrate through a gluing connection, a clamping connection, etc., to form the plurality of culture regions.

In some embodiments, the roughness of the interior of each culture region may be different from the roughness of the exterior of each culture region, and the interior of each culture region may have the regular structure at submicron to nanometers level, such that the adhesion affinity of the interior of each culture region to the culture is greater than the adhesion affinity of the exterior of each culture region to the culture, thereby improving the adhesion of the culture to the interior of each culture region 120. In some embodiments, the surface of the substrate may be hydrophilic, the roughness of the interior of each culture region may be greater than the roughness of the exterior of each culture region, and the surface of the interior of each culture region may have the regular structure at submicron to nanometers level. By combining the roughness difference with the regular structure at submicron to nanometers scale, the adhesion affinity of the interior of each culture region to the culture can be further increased. In some embodiments, the surface of the substrate may be hydrophobic, the roughness of the interior of each culture region may be greater than the roughness of the exterior of each culture region, and the surface of the interior of each culture region may have the regular structure at submicron to nanometers level.

In some embodiments, a material of the substrate 110 (e.g., the base plat 112 or the cover plate 114 configured with the plurality of culture regions) may be a hydrophilic material. In some embodiments, the material of the substrate may include glass, quartz, silicon, mica, polystyrene (PS), polymethyl methacrylate (PMMA), polysulfone (PSU), polycarbonate (PC), polypropylene (PP), polyethylene (PE), polyethylene terephthalate glycol (PETG), low-density polyethylene (LDPE), high-density polyethylene (HDPE), polyethylene terephthalate (PET), polyvinylidene fluoride (PVDF), polytetrafluoroethylene (PTFE), polyethylene glycol (PEG), polyethylene oxide (PEG), polypropylene glycol (PPG), polylactic acid (PLA), polyglycolic acid (PGA), polylacticcoglycollic acid (PLGA), polydimethylsiloxane (PDMS), polyvinyl acetate (PVA), copolymers of cycloolefin (COC), copolymers of polypropylene (COP), polymethylpentene (PMP), polystyrene/butadiene copolymer, polystyrene/acrylonitrile copolymer, cellulose acetate, nitrocellulose, hydroxyethyl methylacrylate (HEMA), polyethersulfone, divinyl glycol polymer, nylon 66, or the like, or any combination thereof. In some embodiments, the material of the substrate itself may have hydrophilicity. In some embodiments, the materials of the substrate may have hydrophilicity after being formed into the substrate through specific operating manners or techniques. By using the hydrophilic material for the substrate, both the interior and the exterior of each culture region 120 may have hydrophilicity, leading to relatively flattened shapes of culture liquid droplets adhering to the interior of each culture region 120 during the culture process. After the flattened droplets are solidified into the gelled state, the flattened droplets may contribute to improving the penetration of the external culture medium (e.g., the culture fluid) into the interior. In some embodiments, the substrate 110 may be made of a transparent material (e.g., the glass) to enable observation of the culture. In some embodiments, the materials of the base plate 112 and the cover plate 114 may be the same or different.

In some embodiments, on the basis that the material of the substrate 110 is the hydrophilic material, after a modification operation is performed on the interior of each culture region, a hydrophilicity of the interior of each culture region may be greater than a hydrophilicity of the exterior of each culture region. In some embodiments, the modification operation may include a plasma operation, a radiation irradiation operation, a corona operation, or the like, or any combination thereof. Merely by way of example, when performing the modification operation, the non-culture region 130 of the substrate 110 may be covered with a covering (e.g., a mask, a baffle, etc.), and then the plurality of culture regions 120 may be modified by a plasma machine or a UV lamp. In some embodiments, the hydrophilicity of the interior of each culture region 120 being greater than the hydrophilicity of the exterior of each culture region 120 may refer to that a hydrophilic lipophilic balance (HLB) value of the interior of each culture region 120 is greater than an HLB value of the exterior of each culture region 120, or a water contact angle of the interior of each culture region 120 is less than a water contact angle of the exterior of each culture region 120. In some embodiments, a surface roughness operation (e.g., the polishing operation) may be performed on the substrate 110 (e.g., the interior of each culture region 120), and then the modification operation may be performed on the polished substrate 110. Alternatively, the modification operation may be performed on the substrate 110, and then the surface roughness operation may be performed on the modified substrate 110. Alternatively, the modification operation and the surface roughness operation may be performed on the substrate 110 simultaneously. In some embodiments, the interior of each culture region may be operated (e.g., etched) to form the regular structure at submicron to nanometers level, and then the modification operation may be performed on the interior of each culture region. Alternatively, the modification operation may be performed on the interior of each culture region, and then the interior of each culture region may be operated (e.g., etched) to form the regular structure at submicron to nanometers level. Through the modification operation, the hydrophilicity of the interior of each culture region may be greater than the hydrophilicity of the exterior of each culture region, which can make the adhesion affinity of the interior of each culture region 120 to the culture droplets be greater than the adhesion affinity of the exterior of each culture region 120 to the culture droplets.

In some embodiments, a material of the substrate 110 (e.g., the base plat 112 or the cover plate 114 configured with the plurality of culture regions 120) may be a hydrophobic material. In some embodiments, the material of the substrate may include glass, quartz, silicon, mica, polystyrene (PS), polymethyl methacrylate (PMMA), polysulfone (PSU), polycarbonate (PC), polypropylene (PP), polyethylene (PE), polyethylene terephthalate glycol (PETG), low-density polyethylene (LDPE), high-density polyethylene (HDPE), polyethylene terephthalate (PET), polyvinylidene fluoride (PVDF), polytetrafluoroethylene (PTFE), polyethylene glycol (PEG), polyethylene oxide (PEO), polypropylene glycol (PPG), polylactic acid (PLA), polyglycolic acid (PGA), polylacticcoglycollic acid (PLGA), polydimethylsiloxane (PDMS), polyvinyl acetate (PVA), copolymers of cycloolefin (COC), copolymers of polypropylene (COP), polymethylpentene (PMP), polystyrene/butadiene copolymer, polystyrene/acrylonitrile copolymer, cellulose acetate, nitrocellulose, hydroxyethyl methylacrylate (HEMA), polyethersulfone, divinyl glycol polymer, nylon 66, or the like, or any combination thereof. In some embodiments, the material of the substrate itself may have hydrophobicity. In some embodiments, the materials of the substrate may have hydrophobicity after being formed into the substrate through specific operating manners or techniques. By using the hydrophobic material for the substrate, both the interior and the exterior of each culture region 120 may have hydrophobicity, leading to approximately spherical shapes of culture liquid droplets adhering to the interior of each culture region 120 during the culture process. After the spherical droplets are solidified into the gelled state, the spherical droplets may contribute to providing a more 3D growth space for the culture, thereby facilitating the culture of a complex structure (e.g., an organoid). In some embodiments, the materials of the base plate 112 and the cover plate 114 may be the same or different.

In some embodiments, on the basis that the material of the substrate 110 is the hydrophobic material, after a modification operation is performed on the interior of each culture region, the interior of each culture region 120 may have hydrophobicity. In some embodiments, the modification operation may include a plasma operation, a radiation irradiation operation, a corona operation, or the like, or any combination thereof. By making the interior of each culture region 120 hydrophilic and the exterior of each culture region 120 hydrophobic through the modification operation, the liquid droplets can be relatively easy to adhere to the interior of each culture region 120. In some embodiments, a surface roughness operation may be performed on the substrate 110 (e.g., the interior of each culture region 120), and then the modification operation may be performed on the polished substrate 110. Alternatively, the modification operation may be performed on the substrate 110, and then the surface roughness operation may be performed on the modified substrate 110. Alternatively, the modification operation and the surface roughness operation may be performed on the substrate 110 simultaneously. In some embodiments, the interior of each culture region may be operated (e.g., etched) to form the regular structure at submicron to nanometers level, and then the modification operation may be performed on the interior of each culture region. Alternatively, the modification operation may be performed on the interior of each culture region, and then the interior of each culture region may be operated (e.g., etched) to form the regular structure at submicron to nanometers level.

In some embodiments, the surface of the substrate may be modified with a hydrophilic substance or a functional group (a hydrophilic functional group). The hydrophilic substance may include collagen, fibronectin, laminin, polylysine, gelatin, hyaluronic acid, chitosan, arginine-glycine-aspartic (RGD) peptides, deoxyribonucleic acid (DNA), lysine, metallic gold, block polyether, or the like, or any combination thereof. The hydrophilic functional group may include a hydroxyl group, a carboxyl group, a carbonyl group, an amino group, a thiol group, a sulfonic acid group, a phosphate group, a quaternary ammonium group, an ether bond, a carboxylic ester group, an amide group, or the like, or any combination thereof. In some embodiments, the hydrophilic substance or the hydrophilic functional group (e.g., a material containing the hydrophilic functional groups described above) may be coated on the surface of the substrate 110. In some embodiments, the substance with the hydrophilic functional group may be used to react with the surface of the substrate 110 to chemically modify the surface of the substrate 110. In some embodiments, the material of the substrate 110 may be hydrophilic or hydrophobic. By performing the hydrophilic modification operation on the surface of the substrate 110, the surface of the substrate 110 may be easy to have hydrophilicity. By adopting the substrate 110 having a hydrophilic surface, both the interior and the exterior of each culture region 120 may have hydrophilicity, leading to relatively flattened shapes of culture liquid droplets adhering to the interior of each culture region 120 during the culture process. After the flattened droplets are solidified into the gelled state, the flattened droplets may contribute to improving the penetration of the external culture medium into the interior. In some embodiments, a surface roughness operation may be performed on the substrate 110 (e.g., the interior of each culture region 120), and then the modification operation may be performed on the polished substrate 110 using the hydrophilic substance and/or the hydrophilic functional group. In some embodiments, the interior of each culture region may be operated (e.g., etched) to form the regular structure at submicron to nanometers level, and then the hydrophilic modification operation may be performed on the interior of each culture region.

In some embodiments, the surface of the substrate may be modified with a hydrophobic substance or a functional group (a hydrophobic functional group). The hydrophobic substance may include albumin, and the hydrophobic functional group may include an alkyl group containing one or more double bonds, a polyoxypropylene group, a long-chain perfluoroalkyl group, a polysiloxane group, a hydroxyl group containing aromatic, ester, ether, amine, or amide group, or the like, or any combination thereof. In some embodiments, the hydrophobic substance or the hydrophobic functional group (e.g., a material containing the hydrophobic functional groups described above) may be coated on the surface of the substrate 110. In some embodiments, the substance with the hydrophobic functional group may be used to react with the surface of the substrate 110 to chemically modify the surface of the substrate 110. In some embodiments, the material of the substrate 110 may be hydrophilic or hydrophobic. By performing the hydrophobic modification operation on the surface of the substrate 110, the surface of the substrate 110 may be easy to have hydrophobicity. By adopting the substrate 110 having a hydrophobic surface, both the interior and the exterior of each culture region 120 may have hydrophobicity, leading to approximately spherical shapes of culture liquid droplets adhering to the interior of each culture region 120 during the culture process. After the spherical droplets are solidified into the gelled state, the spherical droplets may contribute to providing a more 3D growth space for the culture, thereby facilitating the culture of a complex structure. In some embodiments, a surface roughness operation may be performed on the substrate 110 (e.g., the interior of each culture region 120), and then the modification operation may be performed on the polished substrate 110 using the hydrophobic substance and/or the hydrophobic functional group. In some embodiments, the interior of each culture region may be operated (e.g., etched) to form the regular structure at submicron to nanometers level, and then the hydrophobic modification operation may be performed on the interior of each culture region.

In some embodiments, the interior of each culture region 120 may be modified with the hydrophilic substance or the hydrophilic functional group. When the material of the substrate is the hydrophilic material, by performing the hydrophilic modification operation on the interior of each culture region 120, the hydrophilicity of the interior of each culture region 120 may be greater than the hydrophilicity of the exterior of each culture region 120, which can make the adhesion affinity of the interior of each culture region 120 to the liquid droplets be greater than the adhesion affinity of the exterior of each culture region 120 to the liquid droplets. When the material of the substrate 110 is the hydrophobic material, by performing the hydrophilic modification operation on the interior of each culture region 120, the interior of each culture region 120 may have hydrophilicity and the exterior of each culture region 120 may have hydrophobicity, which can make the liquid droplets be relatively easy to adhere to the interior of each culture region 120. In some embodiments, the exterior of each culture region 120 may be modified with the hydrophobic substance or the hydrophobic functional group. By performing the hydrophilic modification operation on the interior of each culture region 120 and performing the hydrophobic modification operation on the exterior of each culture region 120, the hydrophilicity or hydrophobicity of the surface of the substrate 110 can be easily altered. At the same time, the liquid droplets can be easier to adhere to the interior of each culture region 120.

In some embodiments, the hydrophilicity of the interior of each culture region 120 may be greater than the hydrophilicity of the exterior of each culture region 120. In some embodiments, the hydrophilicity of the interior of each culture region 120 being greater than the hydrophilicity of the exterior of each culture region 120 may be achieved through various manners. For example, the modification operation (e.g., the plasma operation, the radiation irradiation operation, and/or the corona operation) may be performed on the interior and or the exterior of each culture region 120, such that the hydrophilicity of the interior of each culture region 120 may be greater than the hydrophilicity of the exterior of each culture region 120. As another example, the hydrophilic modification operation (e.g., modified with the hydrophilic substance or the hydrophilic functional group) may be performed on the interior of each culture region 120, such that the hydrophilicity of the interior of each culture region 120 is greater than the hydrophilicity of the exterior of each culture region 120. As yet another example, the modification operation may be performed on the interior of each culture region 120, and then the hydrophilic modification operation (e.g., modified with the hydrophilic or the hydrophilic functional group) may be performed on the interior of each culture region 120, such that the hydrophilicity of the interior of each culture region 120 is greater than the hydrophilicity of the exterior of each culture region 120.

In some embodiments, there may be no height difference between the interior and the exterior of each culture region 120. In some embodiments, the interior of each culture region 120 may be higher than the exterior of each culture region 120. In some embodiments, the interior of each culture region 120 may be lower than the exterior of each culture region 120. For example, the interior of each culture region 120 may be a certain height (e.g., 1 micrometer, 2 micrometers, 10 micrometers, etc.) lower than the exterior of each culture region 120. By setting the interior of each culture region 120 lower than the exterior of each culture region 120, the culture can better adhere to the plurality of culture regions 120.

It should be noted that the descriptions of the culture device are provided for the purposes of illustration, and are not intended to limit the scope of the present disclosure. For persons having ordinary skills in the art, after understanding the principle of the culture device in the embodiments of the present disclosure, various variations and modifications on the culture device may be conducted under the teaching of the present disclosure. However, those variations and modifications may not depart from the protection of the present disclosure. For example, the roughness operation, the operating to form the regular structure at submicron to nanometers level, the modification operation, the hydrophilic modification operation, the hydrophobic modification operation, or the like, or any combination thereof, can be performed on the surface of the substrate.

Some embodiments of the present disclosure further disclose a culture method. For instance, a culture may be cultured using the culture device 100 described in any embodiment of the present disclosure.

In some embodiments, the culture method may include the following operations.
(1) A culture may be added between the base plate 112 and the cover plate 114 when the base plate 112 and the cover plate 114 are in a first relative position where the base plate 112 and the cover plate 114 are relatively close. In some embodiments, the base plate 112 and the cover plate 114 are in the first relative position when the culture device 100 is in a compressed state. In some embodiments, the culture may be added between the base plate 112 and the cover plate 114 via a tube through the culture inlet 172. In some embodiments, a volume of the added culture may be measured, and the volume may be less than a volume to which the plurality of culture regions 120 in the culture device 100 can adhere. In some embodiments, the first relative position may be a preset relative position. When the base plate 112 and the cover plate 114 are in the first relative position, a volume of a chamber formed between the base plate 112 and the cover plate 114 may be less than a volume that an array of the plurality of culture regions can adhere. In this case, the culture may be added between the base plate 112 and the cover plate 114 until the chamber formed between the base plate 112 and the cover plate 114 is filled.
(2) The base plate and the cover plate may be driven to move relative to each other to a second relative position where the base plate and the cover plate are relatively far, such that the culture automatically adheres to the plurality of culture regions. In some embodiments, the base plate 112 and the cover plate 114 are in the second relative position when the culture device 100 is in an extended state. When the base plate 112 and the cover plate 114 are in the second relative position, culture droplets adhered to the plurality of culture regions of the base plate 112 may detach from being in contact with the cover plate 114, and/or the culture droplets adhered to the plurality of culture regions of the cover plate 114 may detach from being in contact with the base plate 112. In some embodiments, the cover plate 114 may be driven to move relative to the base plate 112 by extending the cover plate 114 through a gripping handle. In some embodiments, the base plate 112 and the cover plate 114 may be driven to move relative to each other through the telescoping mechanism (e.g., a gas control mechanism).

In some embodiments, the culture method may further include driving the base plate 112 and the cover plate 114 to move relative to each other in a rhythmic manner to simulate a regular motion of blood circulation. By simulating the regular motion of the blood circulation, a culture environment within the culture device 100 can be relatively closer to the real environment, thereby improving the culture effect. In some embodiments, the rhythmic relative movement of the base plate 112 and the cover plate 114 may be driven by the telescoping mechanism (e.g., the gas control mechanism).

The possible beneficial effects of the embodiments of the present disclosure may include but not limited to the following: (1) enabling rapid and batch inoculation of cultures, thereby effectively improving the culture efficiency; (2) improving the quality of the cell culture product; (3) forming various shapes of the culture droplets to satisfy different culture requirements; (4) reducing culture costs; (5) making the volumes of individual culture droplets uniform, thereby improving consistency in culture outcomes. It should be noted that different embodiments may have different beneficial effects. In different embodiments, the possible beneficial effects may be any one of the above effects, or any combination thereof, or any other beneficial effects that may be obtained.

The above descriptions are merely preferred embodiments of the present disclosure, and are not intended to limit the scope of the present disclosure. Any modifications, equivalent replacements, improvements, etc., made within the spirit and principles of the present disclosure should be included within the scope of protection of the present disclosure.

## Claims

1. A culture device, comprising a substrate, wherein
a surface of the substrate includes a plurality of culture regions, each of the plurality of culture regions being configured to adhere a culture; and
an adhesion affinity of an interior of each of the plurality of culture regions to the culture is greater than an adhesion affinity of an exterior of each of the plurality of culture regions to the culture.

2. The culture device of claim 1, wherein a roughness of the interior of each of the plurality of culture regions is different from a roughness of the exterior of each of the plurality of culture regions, such that the adhesion affinity of the interior of each of the plurality of culture regions to the culture is greater than the adhesion affinity of the exterior of each of the plurality of culture regions to the culture.

3. The culture device of claim 2, wherein the surface of the substrate is hydrophilic, and the roughness of the interior of each of the plurality of culture regions is greater than the roughness of the exterior of each of the plurality of culture regions.

4. The culture device of claim 3, wherein a polishing or etching operation is performed on the interior of each of the plurality of culture regions, such that the roughness of the interior of each of the plurality of culture regions is greater than the roughness of the exterior of each of the plurality of culture regions.

5. The culture device of claim 4, wherein the etching operation includes at least one of a soft etching operation, a laser etching operation, a plasma etching operation, an electron beam etching operation, or a chemical etching operation.

6. The culture device of claim 3, wherein a surface of the interior of each of the plurality of culture regions has a regular structure at submicron to nanometers level.

7. The culture device of claim 2, wherein the surface of the substrate is hydrophobic, the roughness of the interior of each of the plurality of culture regions is greater than the roughness of the exterior of each of the plurality of culture regions, and a surface of the interior of each of the plurality of culture regions has a regular structure at submicron to nanometers level.

8. The culture device of claim 2, wherein the surface of the substrate is hydrophobic, the roughness of the interior of each of the plurality of culture regions is less than the roughness of the exterior of each of the plurality of culture regions, and a surface of the exterior of each of the plurality of culture regions has an irregular structure.

9. The culture device of claim 8, wherein a polishing operation is performed on the exterior of each of the plurality of culture regions, such that the roughness of the interior of each of the plurality of culture regions is less than the roughness of the exterior of each of the plurality of culture regions.

10. The culture device of claim 1, wherein a surface of the interior of each of the plurality of culture regions has a regular structure at submicron to nanometers level, such that the adhesion affinity of the interior of each of the plurality of culture regions to the culture is greater than the adhesion affinity of the exterior of each of the plurality of culture regions to the culture.

11. The culture device of claim 10, wherein the regular structure at submicron to nanometers level includes a plurality of micro-nano monomers arranged in an array, the plurality of micro-nano monomers including at least one of a micro-nano pillar, a micro-nano tube, a micro-nano cone, or a micro-nano wall.

12. The culture device of claim 11, wherein a diameter or a maximum width of each of the plurality of the micro-nano monomers is less than 1 micron.

13. The culture device of claim 11, wherein a head of each of the plurality of the micro-nano monomers has a mushroom-like shape.

14. The culture device of claim 10, wherein an etching operation is performed on the interior of each of the plurality of culture regions, such that the surface of the interior of each of the plurality of culture regions has the regular structure at submicron to nanometers level.

15. The culture device of claim 14, wherein the etching operation includes at least one of a soft etching operation, a laser etching operation, a plasma etching operation, an electron beam etching operation, or a chemical etching operation.

16. The culture device of claim 1, wherein a material of the substrate includes a hydrophilic material.

17. The culture device of claim 16, wherein after a modification operation is performed on the interior of each of the plurality of culture regions, a hydrophilicity of the interior of each of the plurality of culture regions is greater than a hydrophilicity of the exterior of each of the plurality of culture regions, the modification operation including at least one of a plasma operation, a radiation irradiation operation, or a corona operation.

18. The culture device of claim 1, wherein a material of the substrate includes a hydrophobic material.

19. The culture device of claim 18, wherein after a modification operation is performed on the interior of each of the plurality of culture regions, the interior of each of the plurality of culture regions has hydrophilicity, the modification operation including at least one of a plasma operation, a radiation irradiation operation, or a corona operation.

20. The culture device of claim 1, wherein a material of the substrate includes at least one of glass, quartz, silicon, mica, polystyrene (PS), polymethyl methacrylate (PMMA), polysulfone (PSU), polycarbonate (PC), polypropylene (PP), polyethylene (PE), polyethylene terephthalate glycol (PETG), low-density polyethylene (LDPE), high-density polyethylene (HDPE), polyethylene terephthalate (PET), polyvinylidene fluoride (PVDF), polytetrafluoroethylene (PTFE), polyethylene glycol (PEG), polyethylene oxide (PEG), polypropylene glycol (PPG), polylactic acid (PLA), polyglycolic acid (PGA), polylacticcoglycollic acid (PLGA), polydimethylsiloxane (PDMS), polyvinyl acetate (PVA), copolymers of cycloolefin (COC), copolymers of polypropylene (COP), polymethylpentene (PMP), polystyrene/butadiene copolymer, polystyrene/acrylonitrile copolymer, cellulose acetate, nitrocellulose, hydroxyethyl methylacrylate (HEMA), polyethersulfone, divinyl glycol polymer, or nylon 66.

21. The culture device of claim 1, wherein the surface of the substrate is modified with a hydrophilic substance or a hydrophilic functional group, wherein the hydrophilic substance includes at least one of collagen, fibronectin, laminin, polylysine, gelatin, hyaluronic acid, chitosan, arginine-glycine-aspartic (RGD) peptides, deoxyribonucleic acid (DNA), lysine, metallic gold, or block polyether, and the hydrophilic functional group includes at least one of a hydroxyl group, a carboxyl group, a carbonyl group, an amino group, a thiol group, a sulfonic acid group, a phosphate group, a quaternary ammonium group, an ether bond, a carboxylic ester group, or an amide group.

22. The culture device of claim 1, wherein the surface of the substrate is modified with a hydrophobic substance or a hydrophobic functional group, wherein the hydrophobic substance includes albumin, and the hydrophobic functional group includes at least one of an alkyl group containing one or more double bonds, a polyoxypropylene group, a long-chain perfluoroalkyl group, a polysiloxane group, or a hydroxyl group containing aromatic, ester, ether, amine, or amide group.

23. The culture device of claim 1, wherein the interior of each of the plurality of culture regions is modified with a hydrophilic substance or a hydrophilic functional group.

24. The culture device of claim 23, wherein the exterior of each of the plurality of culture regions is modified with a hydrophobic substance or a hydrophobic functional group.

25. The culture device of claim 1, wherein a hydrophilicity of the interior of each of the plurality of culture regions is greater than a hydrophilicity of the exterior of each of the plurality of culture regions.

26. The culture device of claim 1, wherein the culture device includes a base plate and a cover plate, the base plate and the cover plate are movable with respect to each other; and
the surface of the substrate is an upper surface of the base plate or a lower surface of the cover plate.

27. The culture device of claim 26, wherein the upper surface of the base plate includes the plurality of culture regions; and
the adhesion affinity of the interior of each of the plurality of culture regions to the culture is greater than an adhesion affinity of the lower surface of the cover plate to the culture.

28. The culture device of claim 26, wherein the lower surface of the cover plate includes the plurality of culture regions; and
the adhesion affinity of the interior of the plurality of culture regions to the culture is greater than an adhesion affinity of the upper surface of the base plate to the culture.

29. The culture device of claim 26, wherein the upper surface of the base plate includes at least two culture regions, and the lower surface of the cover plate also includes at least two culture regions.

30. The culture device of claim 29, wherein the at least two culture regions on the upper surface of the base plate are staggered with the at least two culture regions on the lower surface of the cover plate.

31. The culture device of claim 26, wherein a periphery of the base plate is connected to a periphery of the cover plate through an extendable corrugated tube.

32. The culture device of claim 26, wherein a periphery of the base plate is connected to a periphery of the cover plate through a flexible membrane and a support frame.

33. The culture device of claim 32, wherein both ends of the support frame are connected to the base plate and the cover plate, respectively, and the support frame undergoes plastic deformation during a transition from a compressed state to an extended state to support the cover plate.

34. The culture device of claim 26, wherein the base plate or the cover plate is configured with a culture inlet, and the cover plate is configured with a gas inlet and outlet.

35. The culture device of claim 26, wherein an upper surface of the cover plate is configured with a gripping handle.

36. The culture device of claim 26, wherein the culture device further includes a telescoping mechanism, and the telescoping mechanism is configured to drive the base plate and the cover plate to move relative to each other.

37. The culture device of claim 36, wherein the telescoping mechanism includes a gas control mechanism, and the gas control mechanism is configured to inflate gas between the base plate and the cover plate such that the base plate and the cover plate move away from each other.

38. The culture device of claim 26, wherein the culture device further includes a side wall, and the side wall is connected to the base plate to form a culture container, the cover plate are movable with respect to the base plate in the culture container.

39. The culture device of claim 38, wherein a periphery of the cover plate includes a sealing ring, and the cover plate abuts against the side wall through the sealing ring.

40. The culture device of claim 26, wherein a roughness of the interior of each of the plurality of culture regions is different from a roughness of the exterior of each of the plurality of culture regions, such that the adhesion affinity of the interior of each of the plurality of culture regions to the culture is greater than the adhesion affinity of the exterior of each of the plurality of culture regions to the culture.

41. The culture device of claim 40, wherein the upper surface of the base plate or the lower surface of the cover plate is hydrophilic, and the roughness of the interior of each of the plurality of culture regions is greater than the roughness of the exterior of each of the plurality of culture regions.

42. The culture device of claim 40, wherein the upper surface of the base plate or the lower surface of the cover plate is hydrophobic, the roughness of the interior of each of the plurality of culture regions is less than the roughness of the exterior of each of the plurality of culture regions, and a surface of the exterior of each of the plurality of culture regions has an irregular structure.

43. The culture device of claim 26, wherein a surface of the interior of each of the plurality of culture regions has a regular structure at submicron to nanometers level, such that the adhesion affinity of the interior of each of the plurality of culture regions is greater than the adhesion affinity of the exterior of each of the plurality of culture regions.

44. A culture method, comprising:
using the culture device according to any one of claims 1 to 43 to culture.

45. The culture method of claim 44, wherein the culture device includes a base plate and a cover plate, the base plate and the cover plate are movable with respect to each other, and the substrate surface is the upper surface of the base plate or the lower surface of the cover plate; and
the culture method comprises:
adding a culture between the base plate and the cover plate when the base plate and the cover plate are in a first relative position where the base plate and the cover plate are relatively close; and
driving the base plate and the cover plate to move relative to each other to a second relative position where the base plate and the cover plate are relatively far, such that the culture automatically adheres to the plurality of culture regions.

46. The culture method of claim 45, further comprising:
driving the base plate and the cover plate to move relative to each other in a rhythmic manner to simulate a regular motion of blood circulation.
